# FASCICULE DE BREVET EUROPEEN

(11) **EP 1 845 959 B1**
(45) Date de publication et mention de la délivrance du brevet: **15.04.2015**
(21) Numéro de dépôt: 06709146.2
(22) Date de dépôt: 23.01.2006
(51) Int. Cl.: A61K 9/50

(54) **MEDICAMENT DESTINE A ETRE ADMINISTRE PAR VOIE ORALE COMPRENANT UN INHIBITEUR DE LA CYCLO-OXYGENASE-2, ET SON PROCEDE DE PREPARATION**
ZUR ORALEN VERABREICHUNG VORGESEHENES ARZNEIMITTEL MIT EINEM CYCLOOXYGENASE-2-INHIBITOR SOWIE HERSTELLUNGSVERFAHREN DAFÜR
MEDICAMENT THAT IS INTENDED FOR ORAL ADMINISTRATION, COMPRISING A CYCLOOXYGENASE-2 INHIBITOR, AND PREPARATION METHOD THEREOF

(30) Priorité: 24.01.2005 FR 0500708
(43) Date de publication de la demande: 24.10.2007
(73) Titulaire: Vetoquinol, 70204 Lure Cedex (FR)
(72) Inventeur: MOREAU, Marinette, F-70200 Saint Germain (FR); OSTY, Nicolas, F-70300 Luxeuil (FR)
(74) Mandataire: Bolinches, Michel Jean-Marie
(86) Numéro de dépôt international: PCT/FR2006/000144
(87) Numéro de publication internationale: WO 2006/077334

(56) Documents cités:
- EP-A- 1 122 243
- WO-A-01/45706

## Description

La présente invention concerne un médicament destiné à être administré par voie orale qui comprend un inhibiteur de la cyclo-oxygénase-2 et qui présente une biodisponibilité améliorée, et un procédé de préparation de ce médicament.

De manière connue, de nombreux principes actifs utilisés dans des médicaments administrés par voie orale, tels que des anti-inflammatoires, présentent l'inconvénient d'être constitués de particules solides qui sont peu solubles dans des milieux aqueux, ce qui pénalise la biodisponibilité orale de ces médicaments.

On peut notamment citer à ce sujet les principes actifs à effet anti-inflammatoire thérapeutique et/ou prophylactique appartenant à la famille des inhibiteurs de la cyclo-oxygénase-2 incluant, à titre non limitatif, un grand nombre de pyrazolyl benzènesulfonamides substitués, telles que le Celecoxib et le Deracoxib (voir le document de Brevet US-A-5 466 823), des isoxazolyl benzènesulfonamides substitués, tels que le Valdecoxib (voir le document US-A-5 633 272), des (méthylsulfonyl)phényl furanones, telles que le Rofecoxib (voir les documents US-A-5 474 995 et US-A-5 981 576), des pyridines substituées, telles que l'Etoricoxib (voir le document US-A-5 861 419), le 2-(3,5-difluorophényl)-3-[4-(méthylsulfonyl)phényl]-2-cyclopentène-1-one (voir le document EP-A-863 134), des benzopyrannes (voir le document US-A-6 034 256), des pyridazinones substituées (voir le document WO-A-00/24719) et des imidazoles tels que le Cimicoxib (voir le document EP-B-1 122 243).

Le document EP-B-1 122 243 présente en page 11 un médicament destiné à être administré par voie orale, par exemple sous forme de comprimé, qui comprend en son coeur un inhibiteur de la cyclo-oxygénase-2 de type imidazole mélangé à un diluant inerte, à un liant et à un agent lubrifiant, et une pellicule externe prévue pour retarder la désintégration et l'absorption du médicament jusqu'à la zone gastro-intestinale de l'organisme. Cette pellicule externe peut être à base de sucre, de gélatine, d'hydroxypropylcellulose ou d'une résine acrylique.

Etant donné que l'absorption de ces principes actifs dans le tube digestif est limitée, la dose thérapeutique à administrer doit être augmentée pour pallier cet inconvénient. C'est la raison pour laquelle on a cherché récemment à améliorer la biodisponibilité de ces inhibiteurs pour une même dose d'administration. Une des façons les plus simples d'améliorer la biodisponibilité est d'augmenter la solubilité du principe actif. Ce paramètre peut être modifié de différentes manières, par ajout d'agents de solubilisation, de tensioactifs, de cyclodextrine, de polymères hydrophiles, ou bien en modifiant la structure des particules d'inhibiteur et en utilisant des techniques de dispersion solide.

Le document WO-A-03/030876 présente un médicament destiné à être administré par voie orale sous forme d'un comprimé se délitant en bouche, qui comprend une dispersion aqueuse de grains de Valdecoxib à titre d'inhibiteur de la cyclo-oxygénase-2, ces grains étant mélangés à un ou des excipients tels que des saccharides qui sont présents à titre majoritaire dans le médicament, pour l'obtention d'un liquide que l'on sèche par atomisation.

Le document WO-A-01/45706 décrit un médicament destiné à être administré par voie orale comprenant un agglomérat de particules solides et inertes sur lesquelles est pulvérisée une solution de celecoxib dans un mélange polymer hydrophile/eau.

Un inconvénient majeur de ces médicaments administrables par voie orale qui comprennent un inhibiteur de la cyclo-oxygénase-2 réside notamment dans leur biodisponibilité relativement insatisfaisante et variable d'un individu à un autre.

Un but de la présente invention est de remédier à cet inconvénient, et ce but est atteint en ce que la Demanderesse vient de découvrir d'une manière surprenante que la pulvérisation, sur des particules solides inertes à base d'au moins un excipient, d'une solution ou d'une suspension de grains micronisés d'un inhibiteur spécifique de la cyclo-oxygénase-2 dans au moins un polymère hydrophile,

permet d'obtenir un médicament destiné à être administré par voie orale et présentant une biodisponibilité améliorée, en comparaison de celle des médicaments de l'art antérieur incorporant un inhibiteur de la cyclo-oxygénase-2, tel que le Cimicoxib, ce médicament selon l'invention comprenant un agglomérat desdites particules solides qui sont agglomérées par le produit de pulvérisation de cette solution ou suspension.

Selon l'invention, cet inhibiteur spécifique de la cyclo-oxygénase-2 est constitué d'au moins un composé tel que décrit dans le document de Brevet précité EP-B-1 122 243 et répondant à la formule suivante (I), ou bien à celle d'un sel ou d'un solvate de ce composé : 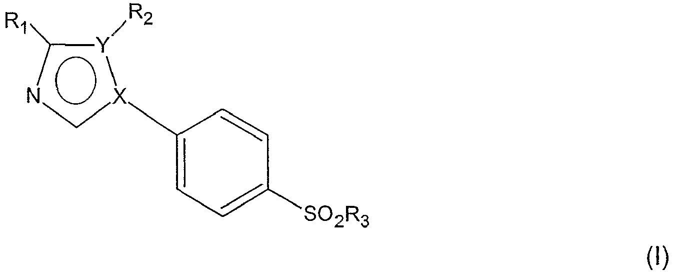 où :
l'un des éléments X et Y représente N et l'autre représente C;
R1 représente un groupe hydrogène, méthyle, halogène, cyano, nitro, -CHO, -COCH₃ ou -COOR₄;
R2 représente un groupe aryl ou hétéroaryl éventuellement substitué par un ou plusieurs groupes choisis indépendamment parmi des groupes halogène, alkyle en C₁₋₈, haloalkyle en C₁₋₈, R₄OC₀₋₈ alkyle, R₄SC₀₋₈ alkyle, cyano, nitro, -NR₄R₆, -NR₄SO₂R₅, -SOR₅, -SO₂R₅, -SO₂NR₄R₆, ou-CONR₄R₆;
R3 représente un groupe alkyle en C₁₋₈, haloalkyle en C₁₋₈ ou-NR₄R₆;
R4 représente un groupe hydrogène, alkyle en C₁₋₈, ou alkyle en C₁₋₈ aryle (où le groupe aryle peut être éventuellement substitué par un ou plusieurs groupes choisis parmi les groupes alkyle en C₁₋₈, halogène, haloalkyle en C₁₋₈, cyano, nitro, R₇OC₀₋₈ alkyle, R₇SC₀₋₈ alkyle, -NR₇R₈,-NR₇COR₅, -COR₇ ou -COOR₇);
R5 représente un groupe alkyle en C₁₋₈ ou haloalkyle en C₁₋₈;
R6 représente un groupe hydrogène, alkyle en C₁₋₈, aryle C₁₋₈ alkyle (où le groupe aryle peut être éventuellement substitué par un ou plusieurs groupes choisis parmi des groupes alkyle en C₁₋₈, halogène, haloalkyle en C₁₋₈, cyano, nitro, R₇OC₀₋₈ alkyle, R₇SC₀₋₈ alkyle, -NR₇R₈,-NR₇COR₅, -COR₇ ou -COOR₇), -COR₈ or -COOR₈;
R7 représente un groupe hydrogène, alkyle en C₁₋₈ ou benzyle;
R8 représente un groupe alkyle en C₁₋₈ ou haloalkyle en C₁₋₈;
le groupe aryle dans les définitions ci-dessus représente un groupe phényle or naphtyle; et
le groupe hétéroaryle dans les définitions ci-dessus représente un groupe pyridine, pyrazine, pyrimidine or pyridazine, qui peuvent éventuellement être fusionnés à un cycle benzène.

A titre encore plus préférentiel, on utilise à titre d'inhibiteur de la cyclo-oxygénase-2 au moins un imidazole, tel que le Cimicoxib, qui répond de manière connue à la formule (II) ci-après :

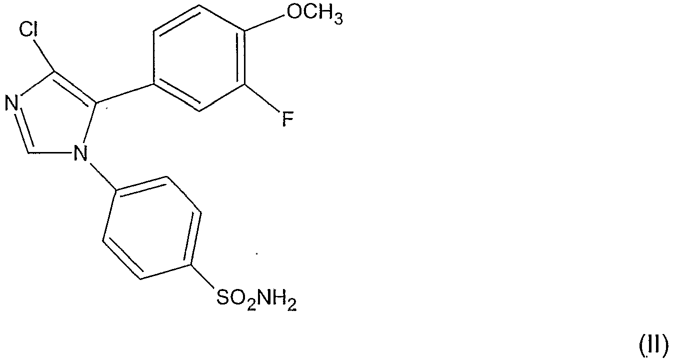

Selon une autre caractéristique avantageuse de l'invention, lesdites particules solides d'excipient(s) sont solubles ou dispersibles dans un milieu aqueux. D'une manière générale, ces particules d'excipient(s) sont hydrophiles, pouvant être de structure cristalline ou amorphe.

De préférence, on utilise à titre d'excipient(s) des particules hydrosolubles ou hydrodispersibles qui sont choisies dans le groupe constitué par les sucres, de préférence le lactose, le saccharose, les hydrolysats de l'amidon tels que la malto-dextrine, la cellulose microcristalline, les sorbitols et les mélanges de plusieurs de ces composés.

A titre encore plus préférentiel, lesdites particules solides comprennent en outre au moins un acide qui est mélangé au(x)dit(s) excipient(s), tel que, préférentiellement, l'acide citrique, ou encore l'acide tartrique ou l'acide fumarique, ce qui permet d'augmenter la solubilité dudit inhibiteur dans l'organisme.

Selon une caractéristique essentielle de l'invention, ledit agglomérat est susceptible d'être obtenu par granulation par voie humide dans un équipement, tel qu'un lit d'air fluidisé.

De préférence, ledit agglomérat selon l'invention comprend spécifiquement le produit de pulvérisation sur lesdites particules solides d'une solution dudit inhibiteur dans le(s)dit(s) polymère(s) hydrophile(s). En effet, la Demanderesse a pu vérifier que, d'une manière inattendue, la dissolution dudit inhibiteur dans ce(s) polymère(s) confère au médicament selon l'invention une biodisponibilité qui est encore améliorée en comparaison de celle conférée par une mise en suspension du même inhibiteur dans le(s) méme(s) polymère(s) hydrophile(s).

Ces grains micronisés dudit inhibiteur sont préférentiellement tels qu'environ 90 % d'entre eux présentent une plus grande dimension en rection transversale qui est inférieure à 20 µm.

De préférence, ledit ou l'un au moins desdits polymère(s) hydrophile(s) est choisi dans le groupe constitué par les polyvinylpyrrolidones, les polyéthylène glycols ou macrogols, les alcools polyvinyliques, les polymères cellulosiques tels que l'hydroxypropylméthylcellulose, l'hydroxypropylcellulose, l'hydroxyméthylcellulose et la carboxyméthylcellulose, les copolymères méthacryliques, l'amidon, les dextrines, la gélatine et les mélanges de plusieurs de ces polymères.

A titre encore plus préférentiel, ledit ou l'un au moins desdits polymère(s) hydrophile(s) est choisi dans le groupe constitué par les polyvinylpyrrolidones et les polyéthylène glycols ou macrogols.

Encore plus préférentiellement, on utilise au moins un polyéthylène glycol ou macrogol de masse moléculaire moyenne en poids Mw allant de 190 à 9 000 g/mol et, à titre encore plus préférentiel, allant de 250 à 600 g/mol et avantageusement de 285 à 420 g/mol.

Selon un mode particulièrement avantageux de réalisation de l'invention, on utilise à titre de polymères hydrophiles, un mélange dudit polyéthylène glycol ou macrogol et d'une polyvinylpyrrolidone de masse moléculaire moyenne en poids Mw allant de 2 000 à 1 000 000 g/mol et, de préférence, allant de 5 000 à 55 000 g/mol.

Selon une autre caractéristique avantageuse de l'invention, ledit produit de pulvérisation de la solution ou suspension dudit inhibiteur dans le(s)dit(s) polymère(s) comprend en outre au moins un tensioactif amphotère, ionique (i.e. anionique ou cationique) ou non ionique.

A titre de tensioactif utilisable, on peut par exemple citer, à titre non limitatif :
- le lauryl sulfate de sodium,
- des esters de sorbitanne polyoxyéthylénés, ou polysorbates,
- les poloxamers.

On peut également utiliser des mélanges de plusieurs de ces tensioactifs. De préférence, on utilise le laurylsulfate de sodium à titre de tensioactif.

Selon une autre caractéristique avantageuse de l'invention, ledit agglomérat comprend ledit inhibiteur selon une fraction massique allant de 1 % à 20 % et, de préférence, allant de 3 % à 10 %.

Avantageusement, ledit agglomérat comprend le(s)dit(s) excipient(s) selon une fraction massique allant de 10 % à 80 % et, de préférence, allant de 30 % à 75 %.

Selon l'invention, ledit agglomérat comprend le(s)dit(s) polymère(s) hydrophile(s) selon une fraction massique allant de 12 % à 25 %.

Egalement à titre avantageux, la fraction massique dudit ou desdits tensioactif(s) dans ledit agglomérat varie de 0,1 % à 6 %.

Selon une autre caractéristique de l'invention, ledit médicament peut optionnellement comporter au moins une couche externe recouvrant ledit agglomérat de particules et comprenant des additifs compatibles choisis dans le groupe constitué par des délitants, des charges, des pigments, des agents d'aromatisation, des tensioactifs, des agents humidifiants, lubrifiants et des mélanges de plusieurs de ces additifs.

Le médicament selon l'invention peut comprendre ladite ou lesdites couche(s) externe(s) selon une fraction massique allant de 0 % à 80 % et, de préférence, allant de 10 % à 50 %.

Avantageusement, le médicament selon la présente invention est constitué d'un dosage solide sous la forme d'un granulé ou d'un comprimé, préférentiellement obtenu par compression dudit agglomérat de particules comprenant optionnellement ladite ou lesdites couche(s) externe(s), ou bien d'une forme solide qui contient ledit agglomérat à l'état de poudre, lequel est logé dans un conditionnement primaire, tel qu'une capsule, une gélule, un sachet ou un flacon.

Des essais ont montré que des médicaments selon l'invention, sous forme d'un comprimé contenant 30 mg de Cimicoxib, sont dissous à au moins 90 % au bout de 30 minutes, dans un milieu à base d'HCl à environ 0,1 N (+ 0,15 % de lauryl sulfate de sodium).

D'autre essais ont montré que des médicaments selon l'invention, sous forme d'un comprimé contenant 10 mg ou bien 5 mg de Cimicoxib, sont dissous à au moins 65 % au bout de 15 minutes, dans un milieu à base de HCl à 0,1 N.

Un procédé de préparation d'un médicament selon l'invention, tel que défini précédemment, comprend les étapes successives suivantes :
(i) la préparation d'un liquide pulvérisable à base de grains micronisés dudit inhibiteur spécifique (cf. formule (I) ci-dessus) de la cyclo-oxygénase-2, notamment un imidazole tel que le Cimicoxib, qui sont en solution ou en suspension dans au moins un polymère hydrophile,
(ii) une pulvérisation dans un granulateur dudit liquide sur des particules solides inertes à base d'au moins un excipient prévu compatible avec ledit inhibiteur, pour l'obtention par granulation par voie humide d'un agglomérat de particules comprenant le produit de pulvérisation de la solution ou suspension desdits grains,
(iii) optionnellement une compression de l'agglomérat de particules obtenu en (ii), et
(iv) optionnellement un recouvrement de l'agglomérat obtenu en (ii) ou en (iii) par au moins une couche externe comprenant des additifs compatibles choisis dans le groupe constitué par des délitants, des charges, des pigments, des agents d'aromatisation, des tensioactifs, des agents humidifiants, des agents lubrifiants et des mélanges de plusieurs de ces additifs.

Selon l'invention, ledit granulateur utilisé à l'étape (ii) est de type à lit d'air fluidisé.

Selon un exemple de réalisation de l'invention, on utilise pour mettre en oeuvre cette granulation par voie humide un granulateur à lit d'air fluidisé fonctionnant à une pression relative allant sensiblement de 1 bar à 1,5 bar, avec une température d'entrée d'air chaud dans ce granulateur allant de 40 à 75° C et une température des particules solides allant de 30 à 50° C.

De préférence, l'étape (i) est mise en oeuvre spécifiquement par dissolution complète dudit inhibiteur dans le(s)dit(s) polymère(s) hydrophile(s), pour l'obtention d'une biodisponibilité améliorée pour le médicament selon l'invention.

On notera que les médicaments selon l'invention sont utilisables pour un traitement thérapeutique et/ou prophylactique d'inflammations diverses d'un organisme humain ou animal, ou de tout autre dysfonctionnement de cet organisme qui est induit par la cyclo-oxygénase-2.

Les caractéristiques précitées de la présente invention, ainsi que d'autres, seront mieux comprises à la lecture de la description suivante de plusieurs exemples de réalisation de l'invention, donnés à titre illustratif et non limitatif.

### EXEMPLE TEMOIN

On a préparé un médicament « témoin » non conforme à l'invention comprenant un comprimé de formule classique constitué :
- d'un noyau à base d'un mélange de particules solides de Cimicoxib et de lactose,
- d'un liquide de granulation contenant de l'eau purifiée et un tensioactif à base d'un polysorbate selon une fraction massique de 0,40 %, pour agglomérer lesdites particules, et
- d'une couche externe formée d'un mélange d'un agent délitant, d'un agent d'aromatisation et d'un agent lubrifiant constitué de stéarate de magnésium.

Plus précisément, la formulation de ce médicament « témoin » était la suivante, pour 100 g :

| | |
|---|---|
| principe actif : Cimicoxib | 8,0 g |
| croscarmelose sodique | 5,0 g |
| amidon prégélatinisé | 15,0 g |
| polysorbate | 0,40 g |
| lactose monohydrate | 68,10 g |
| appétent | 3,0 g |
| stéarate de magnésium | 0,5 g |

On a préparé cet agglomérat de particules « témoin » par granulation par voie humide dans un granulateur à fortes contraintes de cisaillement, et l'on a transformé l'agglomérat de particules obtenu en un comprimé « témoin » par la technique connue de l'homme de l'art.

Ce comprimé « témoin » contient 30 mg de Cimicoxib.

### EXEMPLE 1 SELON L'INVENTION

On a préparé un premier médicament selon l'invention à base d'un agglomérat de particules qui comporte un substrat, à base de particules solides agglomérées par le produit de pulvérisation d'une suspension selon l'invention, et qui est en outre pourvu d'une couche externe.

Cet agglomérat est constitué d'une composition pharmaceutique de formulation suivante (en fractions massiques, couche externe exclue) :
- Substrat :

| | |
|---|---|
| particules de lactose | 72, 90 % |

- Liquide à pulvériser sous forme de suspension :

| | |
|---|---|
| grains micronisés de Cimicoxib | 8,74 % |
| polyvinylpyrrolidone PVP « K25 » | 13,11 % |
| polyéthylène glycol PEG « 400 » | 1,96 % |
| lauryl sulfate de sodium | 3,28 % |

La couche externe recouvrant l'agglomérat de particules présente la formulation suivante (en grammes):

| | |
|---|---|
| Agent délitant « Acdisol » | 5 g |
| Agent d'aromatisation | 3 g |
| Agent lubrifiant (stéarate de magnésium) | 0,5 g |

Dans un premier temps, on a préparé comme suit le liquide pulvérisable à base de Cimicoxib. Tout d'abord, on a dispersé le polymère hydrophile PVP sous agitation. Lorsqu'une solution limpide a été obtenue, on a ajouté l'autre polymère hydrophile PEG. On a ajouté lentement à la solution ainsi obtenue le principe actif micronisé (Cimicoxib) que l'on a alors mélangé sous agitation pendant 30 minutes. Enfin, on a ajouté le laurylsulfate sous agitation pendant 3 minutes.

Dans un second temps, on a pulvérisé la suspension de Cimicoxib ainsi obtenue dans un granulateur à lit d'air fluidisé sur les particules inertes chauffées du substrat (constitué de lactose), sous les conditions suivantes établies pour un lot de 300 g :

| | |
|---|---|
| Pression relative de pulvérisation | 1 bar |
| Température d'entrée d'air chaud | 60° C |
| Température de sortie d'air | 33° C |
| Température des particules | 34° C |
| Durée de la pulvérisation | 36 minutes. |

On a ensuite transformé le granulé ou agglomérat de particules ainsi obtenu en un comprimé, en le recouvrant de la couche externe précitée, ou bien on l'a disposé à l'intérieur d'une capsule, en utilisant dans l'un ou l'autre cas les techniques connues de l'homme de l'art pour obtenir un dosage approprié.

Pour transformer chaque granulé en comprimé et en référence à un lot de 100 g, on a mélangé 91,5 g du granulé obtenu à 8,5 g de la composition de couche externe décrite ci-dessus, en utilisant par exemple un mélangeur de type mélangeur planétaire ou un mélangeur à retournement.

On notera que ce comprimé peut être obtenu au moyen d'une machine à compression alternative ou bien rotative.

### EXEMPLE 2 SELON L'INVENTION

On a préparé un second médicament selon l'invention à base d'un agglomérat de particules qui comporte un substrat, à base de particules solides agglomérées par le produit de pulvérisation d'une autre suspension selon l'invention, et qui est en outre pourvu d'une couche externe.

Cet agglomérat est constitué d'une composition pharmaceutique de formulation suivante (en fractions massiques, couche externe exclue) :
- Substrat :

| | |
|---|---|
| particules de lactose | 61,97 % |

- Liquide à pulvériser sous forme de suspension :

| | |
|---|---|
| grains micronisés de Cimicoxib | 8,74 % |
| polyvinylpyrrolidone PVP « K25 » | 21,86 % |
| polyéthylène glycol PEG « 400 » | 1,97 % |
| lauryl sulfate de sodium | 5,46 % |

La couche externe recouvrant l'agglomérat de particules présente la même formulation que dans l'exemple 1.

On a préparé le liquide pulvérisable, l'agglomérat de particules l'incorporant et le comprimé finalement obtenu à partir de cet agglomérat, en mettant en oeuvre le procédé décrit à l'exemple 1, excepté pour la température de sortie d'air chaud qui est comprise entre 33 et 38° C et pour la température des particules qui est comprise entre 45 et 50° C.

A titre indicatif, on a mesuré par la technique de « HPLC » (chromatographie liquide à haute performance) et dans un milieu d'HCl à environ 0,1 N la solubilité S0 de la poudre de Cimicoxib seule et la solubilité S2 de l'agglomérat de particules selon ce second exemple de l'invention, avec les résultats suivants (solubilités en milligrammes/ litre) :
S0 = 3,1 mg/L et S2 = 26,8 mg/L.

Ce résultat montre que l'agglomérat de particules selon ce second exemple de l'invention présente une solubilité très nettement améliorée en milieu acide.

Le comprimé selon ce second exemple de réalisation de l'invention contient 30 mg de Cimicoxib.

### EXEMPLE 3 SELON L'INVENTION

On a préparé un troisième médicament selon l'invention à base d'un agglomérat de particules qui comporte un substrat, à base de particules solides agglomérées par le produit de pulvérisation d'une autre suspension selon l'invention, et qui est en outre pourvu d'une couche externe.

Cet agglomérat est constitué d'une composition pharmaceutique de formulation suivante (en fractions massiques, couche externe exclue) :
- Substrat :

| | |
|---|---|
| particules de lactose | 53,02 % |
| acide citrique | 16,39 % |
| lauryl sulfate de sodium | 2,73 % |

- Liquide à pulvériser sous forme de suspension :

| | |
|---|---|
| grains micronisés de Cimicoxib | 8,74 % |
| polyvinylpyrrolidone PVP « K25 » | 13,11 % |
| polyéthylène glycol PEG « 400 » | 3,28 % |
| lauryl sulfate de sodium | 2,73 % |

La couche externe recouvrant l'agglomérat de particules présente la même formulation que dans l'exemple 1.

On a préparé, en mettant en oeuvre le procédé décrit à l'exemple 1, le liquide pulvérisable, l'agglomérat de particules l'incorporant et le comprimé finalement obtenu à partir de cet agglomérat, à ceci près que :
- de l'acide citrique et du lauryl sulfate de sodium (en même quantité que dans le liquide pulvérisable) sont incorporés au substrat préchauffé, et que
- la température de sortie d'air chaud est comprise entre 33 et 38° C et la température des particules est comprise entre 45 et 50° C.

Le comprimé selon ce troisième exemple de réalisation de l'invention contient 30 mg de Cimicoxib.

### EXEMPLE 4 SELON L'INVENTION

On a préparé un quatrième médicament selon l'invention à base d'un agglomérat de particules qui comporte un substrat, à base de particules solides agglomérées par le produit de pulvérisation d'une autre suspension selon l'invention, et qui est en outre pourvu d'une couche externe.

Cet agglomérat est constitué d'une composition pharmaceutique de formulation suivante (en fractions massiques, couche externe exclue) :
- Substrat :

| | |
|---|---|
| particules de lactose | 57,61 % |
| acide citrique | 16,39 % |
| lauryl sulfate de sodium | 2,73 % |

- Liquide à pulvériser sous forme de suspension :

| | |
|---|---|
| grains micronisés de Cimicoxib | 4,37 % |
| polyvinylpyrrolidone PVP « K25 » | 13,11 % |
| polyéthylène glycol PEG « 400 » | 3,06 % |
| lauryl sulfate de sodium | 2,73 % |

La couche externe recouvrant l'agglomérat de particules présente la même formulation que dans l'exemple 1.

On a préparé, en mettant en oeuvre à l'identique le procédé décrit à l'exemple 3, le liquide de pulvérisation, l'agglomérat de particules l'incorporant et le comprimé finalement obtenu à partir de cet agglomérat.

### EXEMPLE 5 SELON L'INVENTION

On a préparé un cinquième médicament selon l'invention à base d'un agglomérat de particules qui comporte un substrat, à base de particules solides agglomérées par le produit de pulvérisation d'une solution selon l'invention, mais qui est dépourvu de couche externe, contrairement aux exemples 1 à 4.

Cet agglomérat est constitué d'une composition pharmaceutique de formulation suivante (en fractions massiques) :
- Substrat :

| | |
|---|---|
| particules de lactose | 59,80 % |
| Cellulose microcristalline | 6,65 % |

- Liquide à pulvériser sous forme de solution :

| | |
|---|---|
| grains micronisés de Cimicoxib | 3,30 % |
| polyéthylène glycol PEG « 400 » | 30,25 % |

Dans un premier temps, on a préparé comme suit le liquide à pulvériser. On a ajouté lentement à une solution de PEG « 400 » les grains micronisés du principe actif (Cimicoxib), que l'on mélange alors sous agitation pendant 20 minutes. On obtient une solution de Cimicoxib dans ce PEG.

Dans un second temps, on a pulvérisé la solution ainsi obtenue dans un granulateur à lit d'air fluidisé, sur les particules de substrat inerte chauffé (constitué de lactose additionné de cellulose microcristalline), sous les conditions suivantes établies pour un lot de 200 g :

| | |
|---|---|
| Pression relative de pulvérisation | 1,5 bars |
| Température d'entrée d'air chaud | 70° C |
| Température de sortie d'air | 33° C |
| Température des particules | 34 à 45° C |
| Durée de la pulvérisation | 17 minutes |

A titre indicatif, on a comparé par la technique de « HPLC » et dans un milieu d'HCl à environ 0,1 N la solubilité S5 de cet agglomérat de particules selon ce cinquième exemple de l'invention à la solubilité S0 de la poudre de Cimicoxib seule, avec les résultats suivants (milligrammes/ litre) :
S0 = 3,1 mg/L et S5 = 23,9 mg/L.

Ce résultat montre que le granulé ou agglomérat de particules selon ce cinquième exemple de l'invention présente une solubilité très nettement améliorée en milieu acide.

On a ensuite directement logé ce granulé à l'intérieur d'une gélule.

### EXEMPLE 6 SELON L'INVENTION

On a préparé un sixième médicament selon l'invention à base d'un agglomérat de particules qui comporte un substrat, à base de particules solides agglomérées par le produit de pulvérisation d'une solution selon l'invention, mais qui est dépourvu de couche externe, contrairement aux exemples 1 à 4.

Cet agglomérat est constitué d'une composition pharmaceutique de formulation suivante (en fractions massiques) :
- Substrat :

| | |
|---|---|
| particules de lactose | 62,55 % |
| Cellulose microcristalline | 6,95 % |

- Liquide à pulvériser sous forme de solution :

| | |
|---|---|
| grains micronisés de Cimicoxib | 3,00 % |
| polyéthylène glycol PEG « 400 » | 27,50 % |

On a obtenu cet agglomérat de particules en mettant en oeuvre à l'identique le procédé décrit ci-dessus à l'exemple 5.

On a ensuite directement logé ce granulé à l'intérieur d'une gélule.

### MESURES DE BIODISPONIBILITE DU MEDICAMENT « TEMOIN » ET DE PLUSIEURS MEDICAMENTS SELON L'INVENTION

On a administré par voie orale à quatre chiens (deux mâles et deux femelles) tous de race « Beagle » le comprimé « témoin », les second et troisième comprimés selon l'invention et la gélule selon le cinquième exemple de l'invention, respectivement obtenus aux exemples « témoin », 2, 3 et 5 ci-dessus. Chaque chien a ainsi reçu une même dose de 30 mg de Cimicoxib lors de l'ingestion de ces quatre types de formulations, en espaçant chaque administration d'un intervalle de temps minimal de 6 jours.

On a collecté des échantillons sanguins relatifs à chaque comprimé ou gélule administrés à chacun des quatre chiens, à divers instants suivant chaque administration de ces formes pharmaceutiques, afin de réaliser une analyse de la biodisponibilité de ces produits en termes de concentration Cₘₐₓ (taux plasmatique de Cimicoxib, en µg/mL) et d'aire sous la courbe « AUC » (« Area Under Curve » en µh/mL, calculée sur 10 heures). Ces instants de collecte (exprimés en heures) étaient les suivants :
0 ; 0,25 h ; 0,5 h ; 0,75 h; 1 h ; 1,5 h ; 2 h ; 3 h ; 4 h ; 5 h ; 6 h ; 8 h ; 10 h ; 24 h ; 32 h ; 48 h.

Le tableau 1 ci-après mentionne les résultats moyens obtenus pour le comprimé « témoin », le second et le troisième comprimés selon l'invention et la gélule selon le cinquième exemple de l'invention administrés à l'ensemble des quatre chiens.

**Tableau 1 :**

| Comprimés testés | Tₘₐₓ(h) | Cₘₐₓ (µg/mL) | « AUC » (µg.h/mL, sur 10 h) |
|---|---|---|---|
| Comprimé « témoin » | 2,13 | 0,2825 | 1,341 |
| Second comprimé invention | 2,76 | 0,6279 | 2,606 |
| Troisième comprimé invention | 2,00 | 0,6215 | 2,355 |
| gélule selon le cinquième exemple de l'invention | 1,33 | 1,648 | 5,938 |

Ce tableau montre que les médicaments selon l'invention présentent une assimilation dans l'organisme (i.e. une biodisponibilité) qui est nettement améliorée par rapport à celle du comprimé « témoin », comme cela ressort des valeurs supérieures de concentration Cₘₐₓ et d'aire « AUC ».

Ce tableau montre en outre que la pulvérisation d'un inhibiteur de la cyclo-oxygénase-2 spécifiquement sous forme de solution (i.e. dissous dans le(s) polymère(s) hydrophile(s)) sur les particules solides de substrat inerte améliore encore la biodisponibilité des médicaments selon l'invention par voie orale.

## Revendications

1. Médicament destiné à être administré par voie orale et présentant une biodisponibilité améliorée, ledit médicament comprenant un agglomérat à base de particules solides inertes à base d'au moins un excipient, ledit agglomérat comprenant un inhibiteur de la cyclo-oxygénase-2 et au moins un polymère hydrophile, **caractérisé en ce que** ledit agglomérat comprend le produit de pulvérisation sur lesdites particules d'une solution ou suspension de grains micronisés dudit inhibiteur dans le(s)dit(s) polymère(s), pour agglomérer lesdites particules, et **en ce que** ledit inhibiteur est constitué d'au moins un composé de formule suivante (I) ou bien un sel ou un solvate de ce composé : 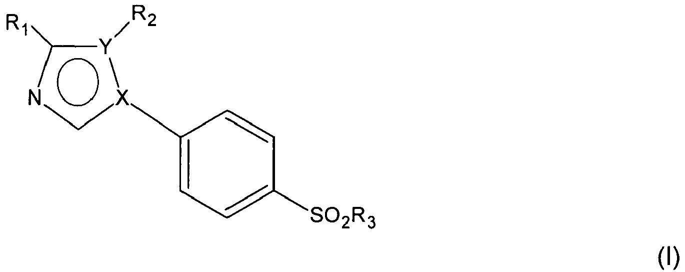 où :
l'un des éléments X et Y représente N et l'autre représente C;
R1 représente un groupe hydrogène, méthyle, halogène, cyano, nitro, -CHO, -COCH₃ ou -COOR₄;
R2 représente un groupe aryl ou hétéroaryl éventuellement substitué par un ou plusieurs groupes choisis indépendamment parmi des groupes halogène, alkyle en C₁₋₈, haloalkyle en C₁₋₈, R₄OC₀₋₈ alkyle, R₄SC₀₋₈ alkyle, cyano, nitro, -NR₄R₆, -NR₄SO₂R₅, -SOR₅, -SO₂R₅, -SO₂NR₄R₆, ou-CONR₄R₆;
R3 représente un groupe alkyle en C₁₋₈, haloalkyle en C₁₋₈ ou - NR₄R₆;
R4 représente un groupe hydrogène, alkyle en C₁₋₈, ou alkyle en C₀₋₈ aryle (où le groupe aryle peut être éventuellement substitué par un ou plusieurs groupes choisis parmi les groupes alkyle en C₁₋₈, halogène, haloalkyle en C₁₋₈, cyano, nitro, R₇OC₀₋₈ alkyle, R₇SC₀₋₈ alkyle, -NR₇R₈, -NR₇COR₅, -COR₇ ou -COOR₇);
R5 représente un groupe alkyle en C₁₋₈ ou haloalkyle en C₁₋₈;
R6 représente un groupe hydrogène, alkyle en C₁₋₈, aryle C₁₋₈ alkyle (où le groupe aryle peut être éventuellement substitué par un ou plusieurs groupes choisis parmi des groupes alkyle en C₁₋₈, halogène, haloalkyle en C₁₋₈, cyano, nitro, R₇OC₀₋₈ alkyle, R₇SC₀₋₈ alkyle, -NR₇R₈,-NR₇COR₅, -COR₇ ou -COOR₇), -COR₈ or -COOR₈;
R7 représente un groupe hydrogène, alkyle en C₁₋₈ ou benzyle;
R8 représente un groupe alkyle en C₁₋₈ ou haloalkyle en C₁₋₈;
le groupe aryle dans les définitions ci-dessus représente un groupe phényle or naphtyle; et
le groupe hétéroaryle dans les définitions ci-dessus représente un groupe pyridine, pyrazine, pyrimidine or pyridazine, qui peuvent éventuellement être fusionnés à un cycle benzène,
ledit médicament étant en outre **caractérisé en ce que** ledit agglomérat comprend le(s)dit(s) polymère(s) hydrophile(s) selon une fraction massique allant de 12% à 25%.

2. Médicament selon la revendication 1, **caractérisé en ce que** ledit produit de pulvérisation de la solution ou suspension dudit inhibiteur dans le(s)dit(s) polymère(s) comprend en outre au moins un tensioactif amphotère, ionique ou non ionique, **en ce que** ledit tensioactif est le laurylsulfate de sodium la fraction massique dudit ou desdits tensioactif(s) dans ledit agglomérat allant de 0,1 % à 6 %.

3. Médicament selon la revendication 1 ou 2, **caractérisé en ce que** ledit ou l'un au moins desdits polymère(s) hydrophile(s) est choisi dans le groupe constitué par les polyvinylpyrrolidones, les polyéthylène glycols ou macrogol, les alcools polyvinyliques, les polymères cellulosiques tels que l'hydroxypropylméthylcellulose, l'hydroxypropylcellulose et la carboxyméthylcellulose, les copolymères méthacryliques, l'amidon, les dextrines, la gélatine et les mélanges de plusieurs de ces polymères..

4. Médicament selon la revendication 3, **caractérisé en ce que** ledit ou l'un au moins desdits polymère(s) hydrophile(s) est choisi dans le groupe constitué par les polyvinylpyrrolidones et les polyéthylène glycols ou macrogols.

5. Médicament selon la revendication 4, **caractérisé en ce que** ledit ou l'un au moins desdits polyéthylène glycol(s) ou macrogol(s) présente une masse moléculaire moyenne en poids Mw allant de 190 à 9 000 g/mol et de préférence allant de 250 à 600 g/mol..

6. Médicament selon une des revendications 4 ou 5 **caractérisé en ce que** lesdits polymères hydrophiles comprennent un mélange dudit polyéthylène glycol ou macrogol et d'une polyvinylpyrrolidone de masse moléculaire moyenne en poids Mw allant de 2 000 à 1 000 000 g/mol, de préférence allant de 20 000 à 55 000 g/mol.

7. Médicament selon la revendication 1, **caractérisé en ce que** ledit inhibiteur est constitué d'au moins un imidazole, tel que le Cimicoxib.

8. Médicament selon une des revendications précédentes, **caractérisé en ce que** ledit agglomérat comprend ledit inhibiteur selon une fraction massique allant de 1 % à 20 %, de préférence allant de 3 % à 10 %.

9. Médicament selon la revendication 8, **caractérisé en ce que** ledit agglomérat comprend le(s)dit(s) excipient(s) selon une fraction massique allant de 10 % à 80 %, de préférence allant de 30 % à 75 %.

10. Médicament selon une des revendications précédentes, **caractérisé en ce que** le(s)dit(s) excipient(s) comprend ou comprennent des particules inertes hydrosolubles ou hydrodispersibles qui sont choisies dans le groupe constitué par les sucres, de préférence le lactose, le saccharose, les hydrolysats de l'amidon tels que la malto-dextrine, la cellulose microcristalline, les sorbitols et les mélanges de plusieurs de ces composés.

11. Médicament selon une des revendications précédentes, **caractérisé en ce que** ledit agglomérat comprend en outre au moins un acide qui est mélangé auxdites particules d'excipient(s), tel que l'acide citrique, l'acide tartrique ou l'acide fumarique.

12. Médicament selon une des revendications précédentes, **caractérisé en ce qu'**il comporte au moins une couche externe recouvrant ledit agglomérat et comprenant des additifs compatibles choisis dans le groupe constitué par des délitants, des charges, des pigments, des agents d'aromatisation, des tensioactifs, des agents humidifiants, des agents lubrifiants et des mélanges de plusieurs de ces additifs.

13. Médicament selon une des revendications précédentes, **caractérisé en ce qu'**il est constitué dudit agglomérat de particules solides se présentant sous la forme d'une poudre logée dans un conditionnement primaire, ou bien sous la forme d'un comprimé.

14. Procédé de préparation d'un médicament selon une des revendications précédentes, **caractérisé en ce qu'**il comprend les étapes successives suivantes :
(i) la préparation d'un liquide pulvérisable à base de grains micronisés dudit inhibiteur de la cyclo-oxygénase-2, notamment un imidazole tel que le Cimicoxib, qui sont en solution ou en suspension dans au moins un polymère hydrophile,
(ii) une pulvérisation dans un granulateur, ledit granulateur est de type à lit d'air fluidisé, ledit liquide sur des particules solides inertes à base d'au moins un excipient prévu compatible avec ledit inhibiteur, pour l'obtention par granulation par voie humide d'un agglomérat de particules comprenant le produit de pulvérisation de la solution ou suspension desdits grains,
(iii) optionnellement une compression de l'agglomérat de particules obtenu en (ii), et
(iv) optionnellement un recouvrement de l'agglomérat obtenu en (ii) ou en (iii) par au moins une couche externe comprenant des additifs compatibles choisis dans le groupe constitué par des délitants, des charges, des pigments, des agents d'aromatisation, des tensioactifs, des agents humidifiants, des agents lubrifiants et des mélanges de plusieurs de ces additifs.

15. Procédé selon la revendication 14, **caractérisé en ce que** la température d'entrée d'air chaud dans ledit granulateur est comprise entre 40° C et 75° C.

16. Procédé selon une des revendications 14 ou 15, **caractérisé en ce que** la température desdites particules solides dans ledit granulateur est comprise entre 30° C et 50° C.

17. Procédé selon une des revendications 14 à 16, **caractérisé en ce que** l'étape (i) est mise en oeuvre par dissolution complète dudit inhibiteur dans le(s)dit(s) polymère(s).

## Patentansprüche

1. Medikament, das dazu bestimmt ist, auf oralem Weg verabreicht zu werden, und das eine verbesserte Bioverfügbarkeit aufweist, wobei das Medikament ein Agglomerat auf der Basis von inerten festen Partikeln auf der Basis wenigstens eines Exzipiens umfasst, wobei das Agglomerat einen Inhibitor der Cyclooxygenase-2 und wenigstens ein hydrophiles Polymer umfasst, **dadurch gekennzeichnet, dass** das Agglomerat das Pulverisierungsprodukt auf den Partikeln einer Lösung oder Suspension von mikronisierten Körnern des Inhibitors in dem Polymer (den Polymeren), um die Partikel zu agglomerieren, umfasst und dass der Inhibitor aus wenigstens einer Verbindung der folgenden Formel (I) oder auch einem Salz der Solvat dieser Verbindung besteht: 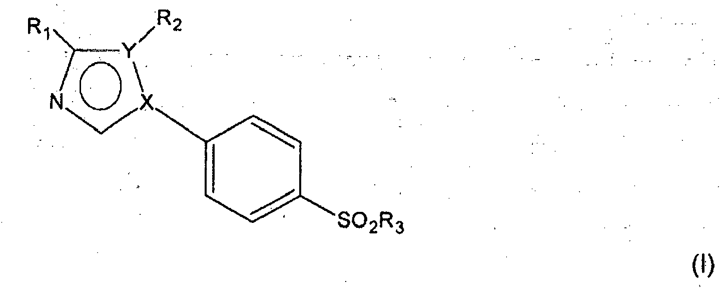 worin:
eins der Elemente X und Y N darstellt und das andere C darstellt;
R1 eine Wasserstoff-, Methyl-, Halogen-, Cyano-, Nitro-, -CHO-, -COCH₃- oder -COOR₄-Gruppe darstellt;
R2 eine Aryl- oder Heteroarylgruppe darstellt, die gegebenenfalls mit einer oder mehreren Gruppen substituiert ist, die unabhängig ausgewählt ist/sind aus den Gruppen Halogen, C₁₋₈-Alkyl, C₁₋₈-Halogenalkyl, R₄OC₀₋₈-Alkyl, R₄SC₀₋₈-Alkyl, Cyano, Nitro, -NR₉R₆, -NR₄SO₂R₅, -SOR₅, -SO₂R₅, -SO₂NR₄R₆ oder -CONR₉R₆;
R3 eine C₁₋₈-Alkyl-, C₁₋₈-Halogenalkyl- oder -NR₄R₆-Gruppe darstellt;
R4 eine Wasserstoff-, C₁₋₈-Alkyl- oder C₀₋₈-Alkylarylgruppe (worin die Arylgruppe gegebenenfalls mit einer oder mehreren Gruppen substituiert sein kann, ausgewählt aus den Gruppen C₁₋₈-Alkyl, Halogen, C₁₋₈-Halogenalkyl, Cyano, Nitro, R₇OC₀₋₈-Alkyl, R₇SC₀₋₈-Alkyl, -NR₇R₈, -NR₇COR₅, -COR₇ oder -COOR₇) darstellt;
R5 eine C₁₋₈-Alkyl- oder C₁₋₈-Halogenalkylgruppe darstellt;
R6 eine Wasserstoff-, C₁₋₈-Alkyl-, Aryl-C₁₋₈-Alkyl- (worin die Arylgruppe gegebenenfalls mit einer Gruppe oder mehreren Gruppen, ausgewählt aus C₁₋₈-Alkyl-, Halogen-, C₁₋₈-Halogenalkyl-, Cyano-, Nitro-, R₇OC₀₋₈-Alkyl-, R₇SC₀₋₈-Alkyl-, -NR₇R₈-, -NR₇COR₅-, -COR₇- oder -COOR₇-Gruppen, substituiert sein kann), -COR₈- oder -COOR₈-Gruppe darstellt;
R7 eine Wasserstoff-, C₁₋₈-Alkyl- oder Benzylgruppe darstellt;
R8 eine C₁₋₈-Alkyl- oder C₁₋₈-Halogenalkylgruppe darstellt;
die Arylgruppe in den obigen Definitionen eine Phenyl- oder Naphtylgruppe darstellt und
die Heteroarylgruppe in den obigen Definitionen eine Pyrridin-, Pyrazin-, Pyrimidin- oder Pyridazingruppe darstellt, die gegebenenfalls mit einem Benzolring kondensiert sein können,
wobei das Medikament außerdem **dadurch gekennzeichnet ist, dass** das Agglomerat das genannte hydrophile Polymer (die genannten hydrophilen Polymere) entsprechend einer Massenfraktion von 12% bis 25% umfasst.

2. Medikament gemäß Anspruch 1, **dadurch gekennzeichnet, dass** das Pulverisierungsprodukt der Lösung oder Suspension des Inhibitors in dem genannten Polymer (den genannten Polymeren) außerdem wenigstens ein amphoteres, ionisches oder nicht-ionisches grenzflächenaktives Mittel umfasst, dass das grenzflächenaktive Mittel Natriumlaurylsulfat ist, die Massenfraktion des grenzflächenaktiven Mittels oder der grenzflächenaktiven Mittel in dem Agglomerat 0,1% bis 6% ist.

3. Medikament gemäß Anspruch 1 oder 2, **dadurch gekennzeichnet, dass** das genannte hydrophile Polymer oder das wenigstens eine der genannten hydrophilen Polymere ausgewählt ist aus der Gruppe, bestehend aus Polyvinylpyrrolidonen, Polyethylenglycolen oder Macrogolen, Polyvinylalkoholen, Cellulosepolymeren wie Hydroxypropylmethylcellulose, Hydroxypropylcellulose und Carboxymethylcellulose, Methacrylcopolymeren, Stärke, Dextrinen, Gelatinen und Gemischen aus mehreren dieser Polymeren.

4. Medikament gemäß Anspruch 3, **dadurch gekennzeichnet, dass** das genannten hydrophile Polymer oder das wenigstens eine der genannten hydrophilen Polymeren ausgewählt ist aus der Gruppe, bestehend aus Polyvinylpyrrolidonen und Polyethylenglycolen oder Macrogolen.

5. Medikament gemäß Anspruch 4, **dadurch gekennzeichnet, dass** das genannten Polyethylenglycol oder Macrogol oder das wenigstens eine der genannten Polyethylenglycole oder Macrogole eine gewichtsmittlere Molekülmasse Mw von 190 bis 9.000 g/Mol und vorzugsweise von 250 bis 600 g/Mol aufweist.

6. Medikament gemäß einem der Ansprüche 4 oder 5, **dadurch gekennzeichnet, dass** die genannten hydrophilen Polymere ein Gemisch des Polyethylenglycols oder Macrogols und eines Polyvinylpyrrolidons mit einer gewichtsmittleren Molekülmasse von 2.000 bis 1.000.000 g/Mol, vorzugsweise von 20.000 bis 55.000 g/Mol, umfassen.

7. Medikament gemäß Anspruch 1, **dadurch gekennzeichnet, dass** der Inhibitor aus wenigstens einem Imidazol besteht, z.B. Cimicoxib.

8. Medikament gemäß einem der vorangehenden Ansprüche, **dadurch gekennzeichnet, dass** das Agglomerat den Inhibitor entsprechend einer Massenfraktion von 1% bis 20%, vorzugsweise von 3% bis 10%, umfasst.

9. Medikament gemäß Anspruch 8, **dadurch gekennzeichnet, dass** das Agglomerat das genannte Exzipiens (die genannten Exzipientien) entsprechend einer Massenfraktion von 10% bis 80%, vorzugsweise von 30% bis 75%, umfasst.

10. Medikament gemäß einem der vorangehenden Ansprüche, **dadurch gekennzeichnet, dass** das genannte Exzipiens (die genannten Exzipientien) wasserlösliche oder wasserdispergierbare inerte Partikel umfasst oder umfassen, die ausgewählt sind aus der Gruppe, bestehend aus Zuckern, vorzugsweise Lactose, Saccharose, Stärkehydrolysaten wie Maltodextrin, mikrokristalliner Cellulose, Sorbit und Gemischen von mehreren dieser Verbindungen.

11. Medikament gemäß einem der vorangehenden Ansprüche, **dadurch gekennzeichnet, dass** das genannte Agglomerat außerdem wenigstens eine Säure umfasst, die mit den Exzipienspartikeln vermischt ist, wie z.B. Citronensäure, Weinsäure oder Fumarsäure.

12. Medikament gemäß einem der vorangehenden Ansprüche, **dadurch gekennzeichnet, dass** es wenigstens eine äußere Schicht umfasst, die das Agglomerat überzieht und kompatible Additive umfasst, die aus der Gruppe, bestehend aus Sprengmitteln, Füllstoffen, Pigmenten, Aromatisierungsmitteln, grenzflächenaktiven Mitteln, Feuchthaltemitteln, Schmiermitteln und Gemischen von mehreren dieser Additive, ausgewählt sind.

13. Medikament gemäß einem der vorangehenden Ansprüche, **dadurch gekennzeichnet, dass** es aus dem genannten Agglomerat fester Partikel besteht, das sich in Form eines Pulvers, das sich in einer ersten Verpackung befindet, oder auch in der Form einer Tablette präsentiert.

14. Verfahren zur Herstellung eines Medikaments gemäß einem der vorangehenden Ansprüche, **dadurch gekennzeichnet, dass** es die folgenden aufeinanderfolgenden Stufen umfasst:
(i) die Herstellung einer pulverisierbaren Flüssigkeit auf der Basis mikronisierter Körner des Inhibitors der Cyclooxygenase-2, insbesondere eines Imidazols wie Cimicoxib, die in Lösung oder in Suspension in wenigstens einem hydrophilen Polymer sind,
(ii) Pulverisierung in einem Granulator, wobei der Granulator vom Wirbelschichttyp ist, die Flüssigkeit auf den inerten festen Partikeln auf der Basis wenigstens eines Exzipiens mit dem genannten Inhibitor kompatibel ist, um durch Granulierung auf feuchtem Weg ein Agglomerat von Partikeln zu erhalten, das das Produkt der Pulverisierung der Lösung oder Suspension der Körner umfasst,
(iii) gegebenenfalls Verpressen des Agglomerats aus Partikeln, das in (ii) erhalten wurde, und
(iv) gegebenenfalls Überziehen des in (ii) oder in (iii) erhaltenen Agglomerats mit wenigstens einer äußeren Schicht, welche kompatible Additive umfasst, die ausgewählt sind aus der Gruppe, bestehend aus Sprengmitteln, Füllstoffen, Pigmenten, Aromatisierungsmitteln, grenzflächenaktiven Substanzen, Feuchthaltemitteln, Schmiermitteln und Gemischen von mehreren dieser Additive.

15. Verfahren gemäß Anspruch 14, **dadurch gekennzeichnet, dass** die Eintrittstemperatur von warmer Luft in den Granulator zwischen 40°C und 75°C liegt.

16. Verfahren gemäß einem der Ansprüche 14 oder 15, **dadurch gekennzeichnet, dass** die Temperatur der festen Partikel in dem Granulator zwischen 30°C und 50°C ist.

17. Verfahren gemäß einem der Ansprüche 14 bis 16, **dadurch gekennzeichnet, dass** die Stufe (i) durch vollständige Lösung des Inhibitors in dem genannten Polymer (den genannten Polymeren) durchgeführt wird.

## Claims

1. Medicament intended for oral administration and which' has improved bioavailability, said medicament comprising an agglomerate based on inert sold particles having a base of at least one excipient, said agglomerate comprising a cyclo-oxygenase-2 inhibitor and at least one hydrophilic polymer, **characterised in that** said agglomerate comprises the product of spraying a solution or suspension of micronised grains of said inhibitor in said polymer(s) onto said particles to agglomerate said particles, and **in that** said inhibitor is made up of at least one compound based on formula (I) or a salt or a solvate of this compound: 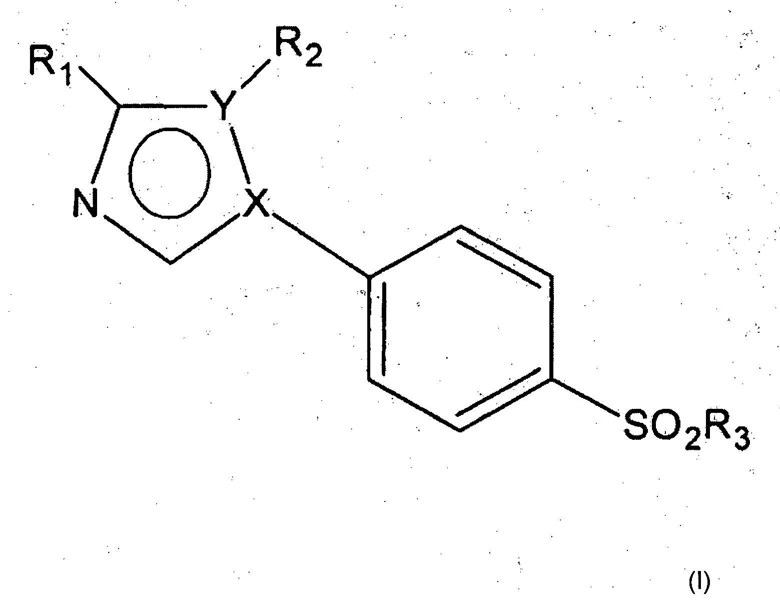 where:
one of the elements X and Y represents N and the other represents C;
R1 represents a hydrogen, methyl, halogen, cyano, nitro, -CHO, -COCH₃ or -COOR₄ group;
R2 represents an aryl or heteroaryl group optionally substituted by one or more groups chosen independently from the halogen, C₁₋₈ alkyl, C₁₋₈ haloalkyl, R₄OC₀₋₈ alkyl, R₄SC₀₋₈ alkyl, cyano, nitro, -NR₄R₆, -NR₄SO₂R₅, -SOR₅, -SO₂R₅, -SO₂NR₄R₆ or -CONR₄R₆ groups;
R3 represents a C₁₋₈ alkyl, C₁₋₈ haloalkyl or NR₄R₆ group;
R4 represents a hydrogen, C₁₋₈ alkyl or C₀₋₈ alkyl aryl group (where the aryl group may optionally be substituted by one or more groups chosen from the C₁₋₈ alkyl, halogen, C₁₋₈ haloalkyl, cyano, nitro, R₇OC₀₋₈ alkyl, R₇SC₀₋₈ alkyl, -NR₇R₈, -NR₇COR₅, -COR₇ or -COOR₇ groups);
R5 represents a C₁₋₈ alkyl or C₁₋₈ haloalkyl group;
R6 represents a hydrogen, C₁₋₈ alkyl, C₁₋₈ aryl alkyl group (where the aryl group may optionally be substituted by one or more groups chosen from the C₁₋₈ alkyl, halogen, C₁₋₈ haloalkyl, cyano, nitro, R₇OC₀₋₈ alkyl, R₇SC₀₋₈ alkyl, -NR₇R₈, -NR₇COR₅, -COR₇ or -COOR₇ groups), -COR₈ or -COOR₈ group;
R7 represents a hydrogen, C₁₋₈ alkyl or benzyl group;
R8 represents a C₁₋₈ alkyl or C₁₋₈ haloalkyl group;
the aryl group in the definitions above representing a phenyl or naphthyl group; and
the heteroaryl group in the definitions above represents a pyridine, pyrazine, pyrimidine or pyridazine group, which may optionally be fused to a benzene ring,
said medicament further being **characterised in that** said agglomerate comprises said hydrophilic polymer(s) in a fraction by weight ranging from 12% to 25 %.

2. Medicament as claimed in claim 1, **characterised in that** said product of spraying the solution or suspension of said inhibitor on said polymer(s) further comprises at least one amphoteric, ionic or non-ionic surfactant, and **in that** said surfactant is sodium lauryl sulphate, the fraction by weight of said surfactant(s) in said agglomerate ranging from 0.1 % to 6%.

3. Medicament as claimed in claim 1 or 2, **characterised in that** said or at least one of said hydrophilic polymer(s) is chosen from the group comprising polyvinyl pyrrolidones, polyethylene glycols or macrogols, polyvinyl alcohols, cellulose polymers such as hydroxypropylmethyl cellulose, hydroxypropyl cellulose and carboxymethyl cellulose, methacrylic copolymers, starch, dextrins, gelatine and mixtures of several of these polymers.

4. Medicament as claimed in claim 3, **characterised in that** said or at least one of said hydrophilicpolymer(s) is chosen from the group comprising polyvinyl pyrrolidones and polyethylene glycols or macrogols.

5. Medicament as claimed in claim 4, **characterised in that** said or at least one of said polyethylene glycol(s) or macrogol(s) has a weight average molecular weight Mw ranging from 190 to 9,000 g/mol and preferably ranging from 250 to 600 g/mol.

6. Medicament as claimed in one of claims 4 or 5, **characterised in that** said hydrophilic polymers comprise a mixture of said polyethylene glycol or macrogol and a polyvinyl pyrrolidone with a weight average molecular weight Mw ranging from 2,000 to 1,000,000 g/mol, preferably ranging from 20,000 to 55,000 g/mol.

7. Medicament as claimed in claim 1, **characterised in that** said inhibitor is at least one imidazole, such as Cimicoxib.

8. Medicament as claimed in one of the preceding claims, **characterised in that** said agglomerate comprises said inhibitor in a fraction by weight ranging from 1 % to 20 %, preferably ranging from 3 % to 10 %.

9. Medicament as claimed in claim 8, **characterised in that** said agglomerate comprises said excipient(s) in a fraction by weight ranging from 10 % to 80 %, preferably ranging from 30 % to 75 %.

10. Medicament as claimed in one of the preceding claims, **characterised in that** said excipient(s) comprises or comprise inert water-soluble or water-dispersible particles which are chosen from the group comprising sugars, preferably lactose, saccharose, starch hydrolysates such as maltodextrin, microcrystalline cellulose, sorbitols and mixtures of several of these compounds.

11. Medicament as claimed in one of the preceding claims, **characterised in that** said agglomerate further comprises at least one acid which is mixed with said particles of excipient(s), such as citric acid, tartaric acid or fumaric acid.

12. Medicament as claimed in one of the preceding claims, **characterised in that** it further comprises an external layer covering said agglomerate and comprising compatible additives chosen from the group comprising disintegrating agents, fillers, pigments, flavourings, surfactants, humectants, lubricants and mixtures of several of these additives.

13. Medicament as claimed in one of the preceding claims, **characterised in that** it is made up of said agglomerate of solid particles in the form of a powder packaged directly in a container or in the form of a tablet.

14. Method of preparing a medicament as claimed in one of the preceding claims, **characterised in that** it comprises the following successive steps:
(i) preparing a sprayable liquid having a base of micronised grains of said cyclo-oxygenase-2 inhibitor, in particular an imidazole such as Cimicoxib, which are dissolved or suspended in at least one hydrophilic polymer,
(ii) spraying said liquid, in a granulator, said granulator being of the fluidised air bed type, onto inert solid particles having a base of at least one excipient compatible with said inhibitor to obtain, by wet granulation, an agglomerate of particles comprising the product of spraying the solution or suspension of said grains,
(iii) optionally compressing the agglomerate of particles obtained in (ii), and
(iv) optionally covering the agglomerate obtained in (ii) or in (iii) with at least one external layer comprising compatible additives chosen from the group comprising disintegrating agents, fillers, pigments, flavourings, surfactants, humectants, lubricants and mixtures of several of these additives.

15. Method as claimed in claim 14, **characterised in that** the intake temperature of hot air in said granulator is between 40°C and 75°C.

16. Method as claimed in one of claims 14 or 15, **characterised in that** the temperature of said solid particles in said granulator is between 30°C and 50°C.

17. Method as claimed in one of claims 14 to 16, **characterised in that** step (i) is implemented by completely dissolving said inhibitor in said polymer(s).
